(19) Europäisches Patentamt
European Patent Office
Office européen des brevets

(11) Veröffentlichungsnummer : **0 378 175 B1**

(12) **EUROPÄISCHE PATENTSCHRIFT**

(45) Veröffentlichungstag der Patentschrift : **31.05.95 Patentblatt 95/22**

(21) Anmeldenummer : **90100378.0**

(22) Anmeldetag : **09.01.90**

(51) Int. Cl.[6] : **G01N 33/563,** G01N 33/577, C12N 15/13, C12N 15/62, C07K 16/46, // C12P21/08, C12N5/18, G01N33/531, G01N33/76, G01N33/68

# (54) Diagnostischer Nachweis unter Verwendung von chimären Antikörpern.

(30) Priorität : **10.01.89 DE 3900534**

(43) Veröffentlichungstag der Anmeldung : **18.07.90 Patentblatt 90/29**

(45) Bekanntmachung des Hinweises auf die Patenterteilung : **31.05.95 Patentblatt 95/22**

(84) Benannte Vertragsstaaten : **AT BE CH DE DK ES FR GB GR IT LI LU NL SE**

(56) Entgegenhaltungen :
**EP-A- 0 266 663**
**EP-A- 0 274 394**
**EP-A- 0 323 806**
**WO-A-86/01533**

(73) Patentinhaber : **BOEHRINGER MANNHEIM GMBH**
**D-68298 Mannheim (DE)**

(72) Erfinder : **Kaluza, Brigitte, Dr.**
**Antdorfer Strasse 12**
**D-8121 Habach (DE)**
Erfinder : **Lenz, Helmut, Dr.**
**von Kühlmann-Strasse 14**
**D-8132 Tutzing (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**EP 0 378 175 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von chimären monoklonalen Antikörpern oder Fragmenten davon für diagnostische Tests.

In der klinischen Diagnose werden seit der Entwicklung der Hybridoma-Technik zur Herstellung von monoklonalen Antikörpern diese in überwiegendem Ausmaß für immunologische Bestimmungen verwendet. Die monoklonalen Antikörper werden hierfür meist in Maus-Zellinien hergestellt und weisen daher, abgesehen von der Antigen-spezifischen hypervariablen Region, nur Maus-spezifische Regionen auf. Solche monoklonalen Antikörper aus Mäusen werden zu immunologischen Bestimmungen, z. B. RIA, IRMA, IEMA verwendet. Bei den sogenannten "sandwich"-Assays, wird in der Diagnose eine im menschlichen Blut oder Serum enthaltene Substanz über zwei Antikörper nachgewiesen, von denen einer an einer Festphase immobilisiert ist, und der zweite eine Markierung trägt. Eine solche Markierung ist normalerweise eine Enzymmarkierung, durch die zum Nachweis eine Farbreaktion ausgelöst werden kann.

In den letzten Jahren wurden bei solchen diagnostischen Nachweisen jedoch immer häufiger falsche überhöhte Testwerte bei bestimmten Patienten festgestellt. Derartige überhöhte Werte kommen dann zustande, wenn zwei Umstände zusammentreffen, nämlich

a) eine "sandwich"-Testkonzeption, die auf einer Verbrückung zweier Antikörper, nämlich eines wandständigen und eines Signal-tragenden Antikörpers durch ein Antigen basiert, und

b) das Auftreten von sogenannten heterophilen Antikörpern oder von Anti-Maus-Antikörpern im Serum des Patienten.

Humane heterophile Antikörper sind gegen IgG verschiedener tierischer Spezies gerichtet, unter anderem gegen IgG der Maus, wodurch in der Regel die Testantikörper betroffen sind. Häufig zeigen heterophile Antikörper jedoch auch Kreuzreaktionen mit IgG von Ratte, Rind, Schaf, Pferd, Meerschweinchen oder Affen, seltener mit IgG von Hund, Katze oder Kaninchen. Ursache hierfür scheinen gemeinsame Epitope im $F(ab')_2$-Fragment (Boscato und Stuart, Clin. Chem. 32 (1988), 1491-1495) oder im Fc-Teil (Clark und Prince, Clin. Chem. 33 (1987), 414) verschiedener IgG-Moleküle zu sein.

Solche heterophilen Antikörper sind in Menschen weit verbreitet und vermutlich durch Umgang mit oder Injektion von tierischen Produkten entstanden. Auch die Entstehung von Anti-Maus-Antikörpern im Menschen ist hauptsächlich darauf zurückzuführen.

Bei den "sandwich"-Assays wird nun auf der einen Seite durch Bindung des polyvalenten heterophilen Antikörpers oder des Anti-Maus-Antikörpers an die monoklonalen Antikörper bereits ohne das Vorliegen des Antigens eine Verbrückung des Nachweis-Antikörpers mit dem Festphasen-Antikörper bewirkt und dadurch ein falsches Testsignal ausgelöst, und zum anderen die korrekte Antigenbindung der Testantikörper sterisch behindert.

Es wurde daher bereits vorgeschlagen, zur Vermeidung derartiger Testbeeinträchtigungen bei "sandwich"-Immunoassays monoklonale Antikörper verschiedener Spezies, z.B. von Maus und Ratte, zu verwenden (Boscato und Stuart, Clin. Chem. 43, (1988) 27-33). Hierdurch kann jedoch das Problem nicht gelöst werden, da wie bereits erwähnt, humane heterophile Antikörper gegen IgG verschiedener tierischer Spezies gerichtet sind. Heterophile Antikörper vieler Patienten reagieren sowohl mit Maus-IgG als auch mit Ratten-IgG. Nachdem monoklonale Antikörper mit hoher Effizienz derzeit nur aus Maus und Ratte gewonnen werden können, sind hier also von vorneherein Einschränkungen gegeben.

Weiter wurde von Boscato und Stuart vorgeschlagen, zur Vermeidung fehlerhafter Meßergebnisse beim Testen von Seren, die heterophile Antikörper enthalten, durch Zusatz von Nicht-Immun-IgG derjenigen Spezies, aus der der monoklonale Test-Antikörper stammt, die Antikörper des Patienten abzusättigen. Die Bereitstellung derartig großer Mengen von Maus-Antikörpern ist jedoch problematisch, teuer und führt offensichtlich nicht zum gewünschten Erfolg.

Von Kahn et al., J. Clin. Endocrin. Metabolism., 66 (1988), 526-533 wurde vorgeschlagen, die im Serum enthaltenen heterophilen Antikörper durch Polyethylenglykolfällung zu beseitigen. Dies ist ebenfalls aufwendig und kann nur für Antigentests, nicht jedoch für Antikörpertests angewandt werden. Weiter wurde in derselben Literaturstelle vorgeschlagen, die diagnostisch einzusetzenden Antikörper zu jodieren, damit sie nicht mehr durch die humanen Anti-Maus-Antikörper erkannt werden können. Dies ist jedoch ebenfalls mit einem großen Aufwand verbunden und führt auf jeden Fall zu einem Aktivitätsverlust der Antikörper.

EP-A-0 323 806 ist am 12.07.89 veröffentlicht und damit Stand der Technik gemäß Artikel 54(3) EPÜ. In dieser Patentanmeldung wird ein für CEA spezifischer chimärer Antikörper beschrieben und dessen Verwendung in ELISA-Verfahren vorgeschlagen.

Ein weiterer Vorschlag, nämlich die heterophilen Antikörper mit einem Anti-Human-Immunglobulin oder mit Protein A zu entfernen (Clin. Chem. 34 (1988), 261-264), ist ebenfalls für Routinebestimmungen zu aufwendig und wiederum nur für Antigentests, jedoch nicht für Antikörpertests geeignet.

Der Erfindung liegt daher die Aufgabe zugrunde, die störenden Auswirkungen von heterophilen Antikörpern in Patientsenserum zu eliminieren und eine störungsfreie quantitative Bestimmung von Substanzen im Patientenserum, sowohl von Antigenen als auch von Antikörpern, zu ermöglichen.

Gelöst wird diese Aufgabe erfindungsgemäß durch die Verwendung mindestens eines chimären monoklonalen Antikörpers, welcher aus einem humanen Antikörper besteht, dessen variable Region ganz oder teilweise durch die entsprechenden Teile eines nicht-humanen monoklonalen Antikörpers der gewünschten Spezifität ersetzt ist, oder eines Fragments davon in einem "Sandwich"- Immunoassay zur quantitativen immunologischen Bestimmung von Substanzen im Blut oder Serum von Patienten, ausgenommen Immunoassays zur Bestimmung von CEA.

Derartige chimäre Antikörper sind aus dem Stand der Technik bekannt, wie etwa von EP-A-0 256 663 oder EP-A-0 274 394, wurden jedoch für andere Verwendungen vorgeschlagen, vor allem für solche, bei denen eine Erhaltung der im $F_c$-Fragment erhaltenen Effektorfunktionen wünschenswert ist und bei denen unerwünschte Nebeneffekte, wie etwa Hypersensibilität, in vivo zu vermeiden sind. Die Probleme, welche durch heterophile Antikörper im Blut von Patienten bei der Immundiagnostik speziell in Sandwich-Immunoassays verursacht werden, sind in keinster Weise genannt oder angedeutet, und eine vorteilhafte Anwendungsmöglichkeit von chimären Antikörpern für Sandwich-Immunoassays ist nicht beschrieben.

Vorzugsweise wird bei der vorliegenden Erfindung ein humaner Antikörper verwendet, bei dem die variablen Regionen ($V_H$) und $V_L$) gegen die entsprechenden Regionen eines nichthumanen monoklonalen Antikörpers (z.B. aus Ratte oder vorzugsweise Maus) ausgetauscht wurden. Besonders bevorzugt wird ein monoklonaler humaner Antikörper verwendet. Ebenfalls bevorzugt wird als ein chimärer Antikörper ein humaner Antikörper verwendet, bei dem die hypervariablen Regionen aus $V_H$ und $V_L$ teilweise - oder besonders bevorzugt - vollständig gegen die entsprechenden Regionen eines nichthumanen monoklonalen Antikörpers (z.B. Ratte und vorzugsweise Maus) ausgetauscht wurden (reshaped antibody). Ein solcher bevorzugter Antikörper enthält also den Fc-Teil, die konstanten Regionen des Fab-Teils, sowie die framework-Bereiche der variablen Regionen eines humanen Antikörpers. Unter "framework-Bereichen" sind die jeweils vier Bereiche der zwei schweren und leichten Ketten eines Antikörpers zu verstehen, die die drei hypervariablen Regionen umgeben, bzw. zwischen ihnen liegen. Der Aufbau solcher chimärer Antikörper ist beispielsweise in Verhoeyen and Riechmann, Bioessay, Vol. 8, 1988, Seiten 74 bis 78 beschrieben. Durch die erfindungsgemäße Verwendung von chimären Antikörpern oder Fragmenten davon wird die Gefahr einer Wechselwirkung der Testantikörper mit heterophilen Antikörpern weitgehend vermieden. Die erfindungsgemäße Verwendung kann für alle Sandwich-Immunoassays unter Verwendung von monoklonalen Antikörpern genutzt werden, beispielsweise auch für RIA, da hierbei ebenfalls eine Verfälschung der Testergebnisse durch heterophile oder Anti-Maus-Antikörper zu befürchten ist. Besonders wichtig und geeignet ist die Erfindung jedoch für Assays wie IRMA und IEMA.

Verfahren zur Herstellung von chimären Antikörpern, bei denen die variablen Bereiche ausgetauscht sind, sind beispielsweise von Brüggemann et al. (1987), J. Exp. Med. 166, 1351-1361, Liu et al. (1987) Proc. Natl. Acad. Sci. USA 84 3439-3443, Whittle et al., Protein Engineering 1 (1987), 499-505, Nature 314 (1985) 452-454, J. Immunol. 137 (1986) 1066-1074, Gene 54 (1987) 33-40 und in der WO86/01533 sowie der EP-A-0 239 400 beschrieben und können vom Fachmann entsprechend der für den speziellen Test erforderlichen Bedingungen abgewandelt werden. Eine leicht abgewandelte Herstellungsmethode für einen chimären Anti-TSH-Antikörper (MAK<TSH>) wird in den Beispielen beschrieben.

Verfahren zur Herstellung von chimären Antikörpern, bei denen nur die hypervariablen Bereiche ausgetauscht sind (reshaped antibody), sind in Nature 321 (1986) 522-525 und Nature 332 (1988) 323-327 beschrieben. Die Herstellung von Fragmenten von Antikörpern kann nach an sich bekannten Methoden erfolgen, wie sie beispielsweise von Johnstone und Thorpe in Immunochemistry in Practice, Blackwell Scientific, 1982, Seiten 52 bis 53, beschrieben sind.

In einer Ausführungsform der Erfindung wird in einem diagnostischen "sandwich"-Immunoassay mindestens ein chimärer monoklonaler Antikörper oder ein Fragment davon verwendet. Hierdurch kann eine Verbrückung der beiden Testantikörper vermieden werden, da höchstens der nicht-chimäre Antikörper von heterophilen Antikörpern oder Maus-Antikörpern gebunden werden kann, für die Verbrückung aber Bindung beider Testantikörper nötig ist. In manchen Fällen, vor allem bei Vorliegen von relativ hohen Anteilen an heterophilen Antikörpern im Patientenserum, kann es jedoch zweckmäßig sein, in immunologischen Tests nur chimäre Antikörper, vor allem zur Vermeidung von Konkurrenzreaktionen, einzusetzen.

Obwohl die heterophilen Antikörper meist an den Fc-Teil der monoklonalen Antikörper binden, ist es bevorzugt, zur Vermeidung jeglicher möglicher Wechselwirkung einen chimären Antikörper zu verwenden, bei dem die variablen oder hypervariablen Regionen eines humanen monoklonalen Antikörpers gegen die entsprechenden Teile eines monoklonalen Maus-Antikörpers der gewünschten Spezifität ausgetauscht sind. Hierdurch wird die Wechselwirkung mit menschlichen Anti-Maus-Antikörpern bzw. heterophilen Antikörpern mit Kreuzreaktivität vermieden.

In einer bevorzugten Ausführungsform der Erfindung wird ein chimärer Anti-TSH-Antikörper oder ein Fragment davon zum Nachweis von Thyroid-stimulierendem Hormon (TSH) im Patientenserum verwendet. Hierfür wurden aus den klonierten Anti-TSH-MAK-cDNA's unter Zuhilfenahme zweier Maus-Immunglobulingene als Rezeptoren und der konstanten Regionen je eines humanen kappa- und gammal-Genes zwei vollständige chimäre Gene für leichte und schwere Kette rekonstruiert, diese in einen Expressionsvektor eingebracht (Beispiel 1) und in einer geeigneten Wirtszelle exprimiert. Der chimäre Anitkörper wurde dann aus dem Nährmedium der Zellen nach an sich bekannten Methoden isoliert.

Erfindungsgemäß gelingt es also, überhöhte Testergebnisse der diagnostischen Immunoassays unter Verwendung von monoklonalen Antikörpern oder Anti-Maus-Antikörpern im Serum oder Blut von Patienten, vor allem bei "sandwich"-Assays, zu verhindern.

Ein weiterer Gegenstand der Erfindung ist somit die Verwendung von chimären monoklonalen Antikörpern, welche aus humanen Antikörpern bestehen, deren variable Regionen ganz oder teilweise durch die entsprechenden Teile von nicht-humanen monoklonalen Antikörpern der gewünschten Spezifität ersetzt sind, oder von Fragmenten davon zur Vermeidung von falsch-positiven Signalen durch unspezifische Bindung zwischen Testantikörpern und heterophilen Antikörpern bei "Sandwich"-Immunoassays zur quantitativen Bestimmung von Substanzen in Blut oder Serum von Patienten.

Ein nochmals weiterer Gegenstand der Erfindung ist ein Verfahren zum diagnostischen Nachweis von Substanzen, ausgenommen CEA, im Serum oder Blut von Patienten durch Bindung zweier gegen die Substanz gerichteter monoklonaler Antikörper $R_1$ und $R_2$, wovon der eine Antikörper $R_1$ an eine feste Phase bindefähig oder bereits gebunden ist und der zweite Antikörper $R_2$ eine Markierung trägt, gegebenenfalls Bindung des Antikörpers $R_1$ an die feste Phase, Abtrennung des gebildeten, festphasengebundenen Komplexes aus $R_1$, Substanz und $R_2$ und Nachweis über die Markierung des Antikörpers $R_2$, welches dadurch gekennzeichnet ist, daß man als mindestens einen der Antikörper $R_1$ oder $R_2$ einen chimären monoklonalen Antikörper, welcher aus einem humanen Antikörper besteht, dessen variable Region ganz oder teilweise durch die entsprechenden Teile eines nicht-humanen monoklonalen Antikörpers der gewünschten Spezifität ersetzt ist, oder ein Fragment davon verwendet.

Unter "bindefähig an eine feste Phase" wird im Rahmen der Erfindung unter anderem auch verstanden, daß der Antikörper $R_1$ über eine zusätzliche Komponente, wie z.B. einen dritten, festphasengebundenen Antikörper, der gegen den Fc-Teil von $R_1$ gerichtet ist, oder ein an $R_1$ gebundenes Biotin-Molekül und festphasengebundenes Streptavidin an der festen Phase verankert werden kann.

Hierbei bestehen beispielhafte Varianten der Testmethode unter anderem in

a) der Verwendung eines festphasengebundenen Antikörpers gegen das Fc-Fragment eines menschlichen Antikörpers, der den Fc-Teil eines chimären humanen, gegen das Antigen gerichteten Antikörpers bindet, und Markierung des Komplexes über einen ebenfalls gegen das Antigen gerichteten, z.B. Peroxidase-markierten zweiten Antikörper;

b) der Verwendung von festphasengebundenem Streptavidin, einem biotinylierten chimären, gegen das Antigen gerichteten Antikörper, sowie einem ebenfalls gegen das Antigen gerichteten markierten zweiten Antikörper;

c) der Verwendung eines festphasengebundenen Antikörpers gegen den Fc$\gamma$-Teil eines Maus-Antikörpers, eines damit bindefähigen, gegen das Antigen gerichteten Maus-Antikörpers und eines gegen das Antigen gerichteten markierten chimären Antikörpers;

d) der Verwendung von festphasengebundenem Streptavidin, einem biotinylierten, gegen das Antigen gerichteten Maus-Antikörper und einem ebenfalls gegen das Antigen gerichteten, markierten chimären Antikörper;

e) der Verwendung eines festphasengebundenen, gegen das Antigen gerichteten chimären Antikörpers und eines ebenfalls gegen das Antigen gerichteten markierten Maus-Antikörpers; oder

f) der Verwendung eines festphasengebundenen, gegen das Antigen gerichteten Maus-Antikörpers und eines ebenfalls gegen das Antigen gerichteten, markierten, chimären Antikörpers.

Die Markierung des einen Antikörpers im erfindungsgemäßen Verfahren kann sowohl eine radioaktive Markierung, als auch eine andere Markierungsart, wie z.B. enzymatische Markierung sein. Bevorzugt wird gemäß der Erfindung eine Enzymmarkierung verwendet, die wiederum über eine, durch dieses Enzym bewirkte Farbreaktion nachgewiesen wird.

Die Markierung kann hierbei auch indirekt über ein an den jeweiligen Antikörper gebundenes Biotin und eine mit Streptavidin verbundene Markierung, z.B. Peroxidase, stattfinden. Als Festphase kommt beispielsweise die Wand eines Reaktionsgefäßes in Frage.

Für alle angegebenen Varianten ist anstelle der Verwendung ganzer Antikörper natürlich wiederum auch die Verwendung von nur bestimmten Fragmenten dieser Antikörper möglich und eine Ausführungsform der vorliegenden Erfindung.

In einer bevorzugten Ausführungsform verwendet man für das erfindungsgemäße Verfahren zwei chimäre monoklonale Antikörper. Hierdurch wird eine weitere Erhöhung der Sicherheit der Testergebnisse, vor allem bei vorliegen von hohen Konzentrationen an heterophilen Antikörpern erreicht.

Die Herstellung der monoklonalen Antikörper kann nach den bereits zitierten, an sich bekannten Methoden durchgeführt werden.

Durch die Erfindung gelingt es, die Beeinträchtigungen von diagnostischen Tests, welche durch heterophile Antikörper oder Anti-Maus-Antikörper im Serum oder Blut von Patienten hervorgerufen werden, auf einfache und wirksame Weise zu vermeiden. Die Notwendigkeit der Zugabe von unspezifischem, Nicht-Immun-IgG der Spezies, aus der die monoklonalen Antikörper gewonnen wurden, bzw. eine aufwendige Fällung der heterophilen oder Anti-Maus-Antikörper, wie dies im Stand der Technik vorgeschlagen wurde, kann erfindungsgemäß vermieden werden und sogar eine deutlich erhöhte Genauigkeit der Testergebnisse erzielt werden.

Die folgenden Beispiele in Verbindung mit den Figuren erläutern die Erfindung weiter.

Fig. 1 zeigt die DNA- und Aminosäuresequenz der kappa-Kette eines monoklonalen Antikörpers gegen TSH;

Fig. 2 zeigt die DNA- und Aminosäuresequenz der gamma-Kette eines monoklonalen Antikörpers gegen TSH;

Fig. 3 zeigt die DNA- und Aminosäuresequenz der kappa-Kette eines chimären monoklonalen Antikörpers gegen TSH;

Fig. 4 zeigt die DNA- und Aminosäuresequenz der gamma-Kette eines chimären monoklonalen Antikörpers gegen TSH;

Fig. 5 zeigt den Vektor p11196, Vektor p11196 wurde auf der Basis der pUC-Vektoren (C. Yanisch-Perron et al., Gene 33, (1985), 103-119) (Nukleotidposition 630 bis 2675 von pUC18) konstruiert. Das NotI-Fragment ( ) trägt die funktionell rearrangierte V-Region eines K-Gens der Maus. Durch Mutagenese (Nukleotidposition 6 bzw. 312) wurde zu Beginn der Beginn der V-Region ein EcoRV und am Ende der J-Region eine XhoI-Schnittstelle eingebracht.

Fig. 6 zeigt den Vektor p11197, Vektor p11197 wurde auf der Basis der pUC-Vektoren (C. Yanisch-Perron et al., Gene 33, (1985), 103-119) (Nukleotidposition 630 bis 2675 von pUC 18) konstruiert. Das NotI-Fragment ( ) trägt die VDJ-Region eines funktionell rearrangierten $\gamma_1$-Gens der Maus.

Fig. 7 zeigt den Vektor p10195, Vektor p10195 wurde auf der Basis von pSV2 neo (R.C. Mulligan und P. Berg (1981), Proc. Nat. Acad. Sci. USA 78, 2072-2076) konstruiert. Zwischen dessen EcoRI und BamHI Schnittstellen wurde ein DNA-Fragment kloniert, welches den K-Enhancer der Maus enthält ( ), sowie ein DNA-Fragment, welches die konstante Region eines menschlichen K-Gens enthält ( ). Dies führte zum Verlust der BamHI-Schnittstelle.

Fig. 8 zeigt den Vektor p11201, Vektor p11201 wurde auf der Basis von pSV2gpt (R.C. Mulligan und P. Berg (1981), Proc. Nat. Acad. Sci. USA 78, 2072-2076) konstruiert. Zwischen dessen EcoRI- und BamHI-Schnittstelle wurde ein DNA-Fragment ( ) kloniert, welches den Enhancer der schweren Immunglobulinketten der Maus enthält, sowie ein DNA-Fragment, welches die konstante Region eines menschlichen $\gamma_1$-Gens enthält ( ). Die EcoRI-Schnittstelle aus pSV2gpt wurde dabei in eine NotI-Schnittstelle umgewandelt.

Fig. 9 und 10 zeigen die Klonierungsschritte der kappa- und gamma-Ketten-Konstruktion;

Fig. 11 zeigt einen Vergleich der Standardkurven für einen Anti-TSH-monoklonalen Antikörper, Kurve 2 (Fig. 1 und 2), und für einen chimären Anti-TSH-monoklonalen Antikörper, Kurve 1 (Fig. 3 und 4, Beispiel 1). OD: optical density, Extinktion

**Beispiel 1**

Herstellung eines chimären Antikörpers gegen TSH (chimärer monoklonaler Antikörper, chimärer Anti-TSH-MAK)

a) Konstruktion von Vektor p11196 (Fig. 5)

Ein 1,8 Kb großes XbaI-Fragment aus dem K-Gen des anti-idiotypischen MAK A2044 (F. Sablitzky et al., (1985), EMBO J. 4, 345-350) wurde an den Enden mit DNA-PolymeraseI-Klenow-Fragment aufgefüllt, mit NotI-Linkern (5'GCGGCCGC3') versehen und in DraII/PvuII gespaltenen pUC18 (Yanisch-Perron, C, G. Vieira und G. Messing, Gene 33 (1985) 103-119) kloniert, dessen Enden ebenfalls aufgefüllt und mit NotI-Linkern versehen worden waren (T. Maniatis, E.F. Fritsch und J. Sambrook (1982), Molecular Cloning - A Laboratory Manual; Cold Spring Harbor Laboratory).

b) Konstruktion von Vektor p11197 (Fig. 6)

Ein 1 Kb großes EcoRI/HindIII-Fragment aus dem γ1-Gen des anti-idiotypischen MAK A2044 (F. Sablitzky et al., (1985), EMBO J. 4, 345-350) wurde an den Enden aufgefüllt, mit NotI-Linkern versehen und in den DraII/PvuII gespaltenen und ebenso behandelten Vektor pUC18 (Yanisch-Perron, C, G. Vieira und G. Messing, Gene 33 (1985) 103-119) kloniert.

c) Oligonukleotid-gerichtete Mutagenesen

In die DNA-Sequenz von Fig. 1 wurde an Position 1 mit Hilfe des Oligonukleotides I eine EcoRV-Schnittstelle und an Position 325 (Oligonukleotid II) eine XhoI-Schnittstelle eingefügt. Die Nukleotide 1 bis 9 des Oligonukleotids I sind zu der Leaderpeptid-Region der kappa-Kette des Anti-TSH-MAK homolog, die nicht in Fig. 1 enthalten ist. Fig. 1 zeigt die reife kappa-Kette des MAK. In die DNA-Sequenz von Fig. 2 wurde an Position 7 (Oligonukleotid III) eine PvuII-Schnittstelle und an Position 344 (Oligonukleotid IV) eine PstI-Schnittstelle eingefügt. In die Sequenz der VJ-Region des K-Gens von MAK A2044 in p11196 (F. Sablitzky et al., (1985), EMBO J. 4, 345-350) wurde an Position 1 (Oligonukleotid V) eine EcoRV-Schnittstelle und an Position 310 (Oligonukleotid VI) eine XhoI-Schnittstelle eingefügt (Fig. 5). Alle Mutagenesen wurden nach der "gapped-duplex"-Methode von Inouye et al., (Synth. Appl. DNA RNA; (1987), Hrsg. S.A. Narang; Academic Press; 181-206), ausgehend von Plasmid-DNA, durchgeführt.

Verwendete Oligonukleotide:

I: 5'ACCAACGGTGATATCGTGATGACCCAG3'

II: 5'GGCACCAAGCTCGAGATCAAACGG3'

III: 5'ATGAGCAGCTGCAAGAGTCAGGAC3'

IV: 5'GTCACCGTCTCTGCAGCCAAAACG3'

V: 5'GATATCGTGCTAACTCAGTCTCCA3'

VI: 5'GCTGCTGGGACCAAGCTCGAGCTG3'

d) Konstruktion von Vektor p10195 (Fig. 7)

In das mit EcoRI und BamHI gespaltene Plasmid pSV2neo (R.C. Mulligan, P. Berg (1981), Proc. Nat. Acad. Sci. USA 78, 2072-2076) wurde ein 2Kb EcoRI/Asp700-Fragment (Asp 700 durch Linker in BamHI umgewandelt) kloniert, welches die Enhancer-Region (Nature 307 (1984) 80-82) des K-Gens von MAK A2044 enthält. In die BamHI-Schnittstelle des resultierenden Plasmides wurde nach Auffüllen der Enden ein 2,8 Kb EcoRI-Fragment (ebenfalls aufgefüllt) aus pC1 (H.-G. Klobeck et al., (1984), Nucl. Acids Res. 12, 6995-7006) kloniert, welches die konstante Region eines menschlichen K-Gens enthält. Eine oberhalb des Maus K-Enhancers gelegene XbaI-Schnittstelle des resultierenden Plasmides wurde durch Linker-Insertion in eine NotI-Schnittstelle umgewandelt (Fig. 7).

e) Konstruktion von Vektor p11201 (Fig. 8)

In das mit EcoRI und BamHI gespaltene Plasmid pSV2gpt (R.C. Mulligan, P. Berg (1981), Proc. Nat. Acad. Sci. USA 78, 2072-2076) wurde ein 7 Kb HindIII-Fragment kloniert, welches die konstanten Regionen eines menschlichen γ1-Gens (γ1-10) enthielt (N. Takahashi et al., (1982), Cell 29, 671-679). (Dieses ursprüngliche HindIII-Fragment wurde durch Auffüllen der HindIII-Enden und Zwischenklonierung in die ebenfalls aufgefüllte Asp718-Schnittstelle von pUC19 (C. Yanisch-Perron et al., (1985), Gene 3, 103-119) in ein EcoRI-BamHI-Fragment umgewandelt). In die EcoRI-Schnittstelle des resultierenden Plasmides wurde in gleichsinniger Orientierung ein 1,6 Kb HindIII-EcoRI-Fragment insertiert, welches die Enhancer-Region (Cell 41 (1985) 885-897) des γ1-Gens von MAK A2044 (F. Sablitzky et al., (1985), EMBO J. 4, 345-350) enthielt. Die HindIII-Schnittstelle wurde dabei mit Hilfe eines Adaptors (5'AATTGCGGGCCGC3' hybridisiert mit 5'AGCTGCGGCCGC3') sowohl in eine NotI-Schnittstelle umgewandelt als auch an die EcoRI-Schnittstelle angepaßt (Fig. 8).

f) Zusammenfügen des Expressionsplasmides für die leichte Kette des chimären Antikörpers

Die DNA von Fig. 1 wurde an den durch Mutagenese eingeführten Schnittstellen mit EcoRV und XhoI restringiert. Das der V-Region entsprechende 323 bp Fragment wurde in den ebenfalls mit EcoRV und XhoI restringierten Vektor p11196 kloniert. Der resultierende Vektor p11223 wurde mit NotI restringiert; das dem Immunglobulin-Anteil entsprechende 1,8 Kb-Fragment wurde isoliert und in den mit NotI restringierten Vektor p10195 kloniert (Fig. 9). Der resultierende Vektor p11225, DSM 5094, wurde durch CsCl-Dichtegradientenzentrifugation (T. Maniatis et al., (1982), Molecular Cloning - A Laboratory Manual; Cold Spring Harbor Laboratory) gereinigt.

g) Zusammenfügen des Expressionsplasmides für die schwere Kette des chimären Antikörpers

Die cDNA von Fig. 2 wurde an den durch Mutagenese eingeführten Schnittstellen mit PvuII und PstI restringiert. Infolge einer internen PstI-Schnittstelle entstehen zwei der V-Region entsprechende Fragmente von 231 bp und 103 bp. Beide Fragmente wurden in ursprünglicher Orientierung in den ebenfalls mit PvuII und PstI restringierten Vektor p11197 kloniert. Der resultierende Vektor p11221 wurde mit NotI restringiert. Das dem Immunglobulinanteil entsprechende 1 Kb große NotI-Fragment wurde in die NotI-Schnittstelle von Vektor p11201 kloniert (Fig. 10). Der resultierende Vektor p11224, DSM 5093, wurde durch CsCl-Dichtegradientenzentrifugation gereinigt (T. Maniatis et al., (1982), Molecular Cloning - A Laboratory Manual; Cold Spring Harbor Laboratory).

h) Transfektion von Säugerzellen mit den Expressionsplasmiden

Sowohl Vektor p11224, DSM 5093, als auch Vektor p11225, DSM 5094, wurden an ihrer einzigen PvuI-Schnittstelle (im bakteriellen amp$^R$-Gen) linearisiert. Sp2/0 Ag14-Zellen (ATCC CRL1581) wurden auf RPMI-Medium (G.E. Moore, R.E. Gerner & H.A. Franklin (1967) Culture of Normal Human Leucocytes. J.A.M.A. 199, 519-526) angezogen, das mit 10% fötalem Kälberserum, 20 mmol/l Glutamin, 1 mmol/l NaPyruvat, 20 µg/ml 8-Azaguanin und Aminosäuren (MEM Aminosäuren, Zusammensetzung vgl. R.G. Ham, (1963) Exp. Cell. Res. 29, 515-526) supplementiert war. Exponentiell wachsende Zellen (ca. 5 x $10^5$/ml) wurden ad 1,25 x $10^6$/ml in 1 x HeBS-Puffer (G. Chu et al., (1987), Nucl. Acids Res. 15, 1311-1326) resuspendiert. Linearisierter Vektor p11224 und p11225, DSM 5094, wurde in einer Konzentration von je 12,5 µg/ml zugegeben. Zellen und DNA wurden für 10 Minuten bei 0°C vorinkubiert, anschließend in 800 µl Aliquoten einem einmaligen elektrischen Puls von 230 V (Bio Rad Gene Pulser) ausgesetzt und danach weitere 10 Minuten bei 0°C inkubiert. Daraufhin wurden die Zellen in RPMI (wie beschrieben, jedoch ohne 8-Azaguanin) auf Klonierungsplatten plattiert.

Ab 24 Stunden nach Transfektion wurde mit G418 (Geneticin Gibco, 1 mg/ml) oder G418 und zusätzlich Mycophenolsäure (in drei Stufen von 250 ng/ml über 500 ng/ml auf 2 µg/ml ansteigend) selektiert. Mit beiden Selektionsprinzipien wurden Klone erhalten, die sowohl Vektor p11224, DSM 5093, als auch Vektor p11225, DSM 5094, enthielten und aktiven chimären Anti-TSH MAK, (ECACC 88091403) exprimierten. Die Aminosäure- und DNA-Sequenz der kappa- und gamma-Kette des chimären Anti TSH-MAK ist in Fig. 3 und 4 dargestellt.

Die Hybridomazellinie, welche den chimären Anti-TSH MAK produziert, ist bei der European Collection of Animal Cell Culture, Porton Down, GB unter der Nummer ECACC 88091403 hinterlegt.

In analoger Weise können folgende chimäre MAK's hergestellt werden:

| ausgehend von der c-DNA eines MAK (Maus) aus Zellinien | chimärer MAK |
|---|---|
| ECACC 84122006 | gegen Follikelstimulierendes Hormon (FSH) |
| ECACC 84122001 | gegen lutenisierendes Hormon (LH) |
| ECACC 85121701 | gegen Testosteron |

**Beispiel 2**

Funktionsnachweis für den chimären Anti-TSH MAK in einem Doppel-MAK-Sandwich-Test.

a) Anti-TSH MAK

Als Anti-TSH-MAK wurde ein MAK verwendet, dessen Aminosäuresequenzen der kappa- und gamma-Ketten in Fig. 1 und 2 dargestellt sind.

b) Herstellung von Peroxidase-Konjugat von einem zu dem Anti-TSH-MAK (Fig. 1 und 2) komplementären TSH-bindenden Maus-Antikörper.

Die IgG-Fraktion des monoklonalen Anti-TSH-Antikörpers, ECACC 87122202, wurde aus Ascites-Flüssigkeit durch Ammonsulfat-Fällung und Chromatographie an DEAE-Ionenaustauscher nach A. Johnstone und R. Thorpe, Immunochemistry in Practice, Blackwell Scientific, 1982, Seiten 44 bis 45, gereinigt. Aus 200 mg IgG wurden 90 mg Fab-Fragmente nach der Methode von Johnston und Thorpe ibid.

Seiten 52 bis 53, hergestellt. 50 mg der Fab-Fragmente wurden mit S-Acetyl-thio- bernsteinsäureanhydrid umgesetzt und mit 50 mg vorpolymerisierter, Maleinimido-hexanoyl-peroxidase gekoppelt, nach der in DE-A-38 25 735, Beispiel 8.2, beschriebenen Methode. Durch AcA 22-Chromatographie wurde ein Konjugatpool mit einem Molekulargewicht von 2 bis 3 Millionen erhalten. Ausbeute 31 mg Protein mit 13500 U Peroxidase-(POD)-Aktivität.

c) Durchführung des Funktionstests

1. Referenz-ELISA (enzyme linked immunosorbent assay) mit Anti-TSH-MAKs

Schritt 1:

Vertiefungen einer Mikrotiterplatte wurden mit 5 µg/ml polyklonalem Schaf(anti Maus Fcγ) IgG (immunosorbiert) durch spontane Adsorption beschichtet (zwei Stunden, 25°C) und mit 1 % protein C in 50 mM HEPES-Puffer/150 mM NaCl, pH 7,0, nachbeladen (30 Minuten bei 25°C) zur Absättigung von restlichen Adsorptionsstellen.

Schritt 2:

In die entleerten Vertiefungen wurde je 0,2 ml Anti-TSH-MAK (Beispiel 2a) in einer Konzentration von 50 ng IgG/ml, gelöst in RPMI-Zellkulturmedium, pipettiert und 60 Minuten bei Raumtemperatur inkubiert. Für Kontrollen wurde Puffer ohne MAK-IgG eingesetzt.

Schritt 3:

Nach dreimaligem Waschen mit Puffer A wurden je 40 µl TSH-Standardlösung bzw. Patientenserum (mit Störfaktoren, 1 + 1 in Puffer B verdünnt) zusammen mit 200 µl puffer B pro Vertiefung pipettiert und 60 Minuten bei Raumtemperatur inkubiert.

Die TSH-Standardlösungen wurden hergestellt durch Aufstocken von Rinderserum mit einer Lösung von TSH. Die sich ergebenden TSH-Konzentrationen der verschieden aufgestockten Standardlösungen wurden durch Messung mit einem kommerziellen TSH-Test (Enzymun-Test$^R$ TSH, Boehringer Mannheim GmbH, Best.Nr. 736 082) zugewiesen.

Schritt 4:

Nach zweimaliger Waschung mit Puffer A wurde in jede Vertiefung 0,2 ml Enzymkonjugatlösung (Beispiel 2b) mit 100 mU/ml POD-Aktivität gefüllt (Verdünnung des Konjugatkonzentrats aus b) in Puffer C). Das Konjugat wurde 60 Minuten bei Raumtemperatur inkubiert.

Schritt 5:

Nach dreimaligem Waschen mit Puffer C wurde jede Vertiefung mit 0,2 ml ABTS®-Substrat (2,2′-Azino-di-[3-ethylbenzthiazolin-sulfonat] 1,9 mmol/l ABTS®, 100 mmol/l Phosphat-Citrat-Puffer, pH 4,4, Natriumperborat 3,2 mmol/l) gefüllt und 60 Minuten bei Raumtemperatur inkubiert.

Die Extinktionswerte der sich ergebenden Farblösungen in den Vertiefungen wurden mit einem, für Mikrotiterplatten geeigneten Photometer bei 405 nm gemessen.

Puffer A:

16 mmol/l Kalium-Phosphatpuffer, pH 6,9.
0,2 % Rinderserumalbumin
0,15 mol/l NaCl
0,1 % Rinder-IgG

Puffer B:

Puffer A mit 0,1 % Schaf-IgG-Zusatz.

Puffer C:

36 mmol/l Kalium-Phosphatpuffer, pH 6,9,
0,2 % Rinderserumalbumin
0,15 mol/l NaCl
2 % Dextran T500
0,01 % Phenol

(Konzentrationen sind Endkonzentrationen im Test).

2. ELISA-Test mit chimärem Anti-TSH-MAK (Beispiel 1)

Die Testdurchführung mit dem chimären MAK folgte genau den Schritten wie im Referenztest c)1. mit folgenden spezifischen Änderungen:

In Schritt 1 wurde polyklonales Schaf(anti Human-Fcγ)-IgG (immunosorbiert) beschichtet. In Schritt 2 wurde eine Verdünnung von Zellkultur-Überstand mit 50 ng chimärem Anti-TSH-MAK/ml pipettiert. Die Konzentration an chimärem Anti-TSH-MAK wurde mit einem Human-Fcγ-spezifischen Mikrotiter-ELISA bestimmt.

Fig. 11 zeigt den Vergleich der Standardkurven für den Test mit Anti-TSH-MAK (Beispiel 2c1) (Kurve 2) und den Test mit chimärem Anti-TSH-MAK (Beispiel 2c2) (Kurve 1). Der Eichkurvenverlauf ist im Rahmen der Fehlergrenzen identisch. Daraus folgt, daß die TSH-Bindungsstelle im chimären MAK unverändert ist und sich der chimäre MAK im Fcγ-Teil wie in humanes IgG verhält.

Als Kontrolle wurde beim Test nach Beispiel 2c2 eine nach Beispiel 2c1 mit Schaf(anti Maus Fcγ)IgG-beschichtete Mikrotiterplatte verwendet. Es ergaben sich erwartungsgemäß keine vom Leerwert verschiedenen Extinktionen für die Standardproben.

In Tabelle 1 sind die Meßwerte für drei verschiedene Patientenseren gezeigt (PS 71, PS 92, PS 99), von denen bekannt war, daß sie in Maus-Doppel-MAK-Immuntests falsch erhöhte Werte ergaben. Als Kontrolle wurde die Probe PS107 mitgetestet, ein normales Humanserum mit <0,5 μU TSH/ml, ohne Störfaktoren. Die sich in beiden Testsystemen ergebenden Extinktionen für die Serumproben wurden an der jeweiligen Standardkurve in μU TSH/ml Probe umgerechnet.

Zur Kontrolle, daß in Humanseren mit Störfaktoren im Testsystem mit chimärem Anti-TSH-MAK eine definierte Menge TSH richtig wiedergefunden wird, wurde jedes der Seren mit 20 μU TSH/ml aufgestockt und ebenfalls gemessen.

## Tabelle 1

Ergebnisse von Funktionstests mit Anti-TSH-MAK und chimärem Anti-TSH-MAK

1. Testsystem mit Schaf (anti Maus Fcγ)-Beschichtung

| Probe | Anti-TSH-MAK (Beispiel 2c1) | Extinktion $\Delta E_{405}$ (gegen Leerwert) | µU TSH/ml |
|---|---|---|---|
| PS 71 | + | 0,585 | 19,2 |
| | - | 0,015 | |
| PS 92 | + | 0,268 | 8,0 |
| | - | 0,008 | |
| PS 99 | + | 0,550 | 17,6 |
| | - | 0,022 | |
| Normalserum 107 | + | 0,015 | <0,5 |
| | - | 0,005 | |

2. Testsystem mit Schaf (anti Human Fcγ) Beschichtung

| Probe | chimärer Anti-TSH-MAK (Beispiel 2c2) | Extinktion $\Delta E_{405}$ | µU/TSH/ml |
|---|---|---|---|
| PS 71 | + | 0,00 | 0,5 |
| | - | 0,00 | |
| PS 92 | + | 0,045 | 1,2 |
| | - | 0,00 | |
| PS 99 | + | 0,015 | <1,0 |
| | - | 0,01 | |
| PS 71 + 20 µU TSH/ml | + | 0,603 | 19,4 |
| PS 92 + 20 µU TSH/ml | + | 0,678 | 22,1 |
| PS 99 + 20 µU TSH/ml | + | 0,641 | 20,8 |
| Nullstandard + 20 µU TSH/ml | + | 0,622 | 20,2 |

Die in den Patientenseren enthaltenen TSH-Konzentrationen wurden mit Enzymun® TSH-Testpackung (Boehringer Mannheim GmbH, Best.Nr. 7 36082) bestimmt. Dieser Test wird nicht gestört durch anti-Maus-IgG-Störfaktoren, weil anstelle eines Konjugats aus einem Fab-Fragment eines Anti-TSH-Antikörpers aus Maus und Peroxidase ein Konjugat aus einem Fab-Fragment aus polyklonalem Anti-TSH-Antikörper aus Schaf und Peroxidase enthalten ist.

Gefundene Werte: PS 71 (0,52 µU/ml), PS 92 (2,2 µU/ml), PS 99 (0,9 µU/ml).

Aus den Werten der Tabelle ist ersichtlich: das Testsystem nach Beispiel 2c1 findet in den Patientenseren mit Störfaktoren massiv falsch erhöhte, scheinbare TSH-Gehalte. Durch Verwendung des chimären Anti-TSH-MAKs in einem sonst völlig analogen Testsystem (Beispiel 2c2) wird diese Störung vollständig beseitigt. Echt erhöhte TSH-Gehalte werden im Rahmen der Fehlergrenze richtig wiedergefunden.

**Patentansprüche**

1. Verwendung mindestens eines chimären monoklonalen Antikörpers, welcher aus einem humanen Antikörper besteht, dessen variable Region ganz oder teilweise durch die entsprechenden Teile eines nicht-

humanen monoklonalen Antikörpers der gewünschten Spezifität ersetzt ist, oder eines Fragments davon in einem "Sandwich"-Immunoassay zur quantitativen immunologischen Bestimmung von Substanzen im Blut oder Serum von Patienten, ausgenommen Immunoassays zur Bestimmung von CEA.

**2.** Verwendung nach Anspruch 1,
**dadurch gekennzeichnet,**
daß der diagnostische Test als immunoradiometrischer (IRMA) oder immunoenzymatischer (IEMA) Assay durchgeführt wird.

**3.** Verwendung nach einem der Ansprüche 1 oder 2,
**dadurch gekennzeichnet,**
daß man einen chimären Antikörper, der die variablen Regionen eines monoklonalen Maus-Antikörpers der gewünschten Spezifität, sowie die konstanten Regionen des Fab-Teils und den Fc-Teil eines monoklonalen humanen Antikörpers enthält, oder ein Fragment davon verwendet.

**4.** Verwendung nach einem der Ansprüche 1 oder 2,
**dadurch gekennzeichnet,**
daß man einen chimären monoklonalen Antikörper, der die hypervariablen Regionen eines monoklonalen Maus-Antikörpers der gewünschten Spezifität und den Fc-Teil, die konstanten Regionen des Fab-Teils sowie die framework-Bereiche aus den variablen Regionen eines humanen monoklonalen Antikörpers enthält, oder ein Fragment davon verwendet.

**5.** Verfahren zum diagnostischen Nachweis von Substanzen, ausgenommen CEA, im Serum oder Blut von Patienten durch Bindung zweier gegen die Substanz gerichteter monoklonaler Antikörper $R_1$ und $R_2$, wovon der eine Antikörper $R_1$ an eine feste Phase bindefähig oder bereits gebunden ist und der zweite Antikörper $R_2$ eine Markierung trägt, gegebenenfalls Bindung des Antikörpers $R_1$ an die feste Phase, Abtrennung des gebildeten, festphasengebundenen Komplexes aus $R_1$, Substanz und $R_2$ und Nachweis über die Markierung des Antikörpers $R_2$,
**dadurch gekennzeichnet,**
daß man als mindestens einen der Antikörper $R_1$ oder $R_2$ einen chimären monoklonalen Antikörper, welcher aus einem humanen Antikörper besteht, dessen variable Region ganz oder teilweise durch die entsprechenden Teile eines nicht-humanen monoklonalen Antikörpers der gewünschten Spezifität ersetzt ist, oder ein Fragment davon verwendet.

**6.** Verfahren nach Anspruch 5,
**dadurch gekennzeichnet,**
daß man als beide Antikörper $R_1$ und $R_2$ chimäre monoklonale Antikörper oder ein Fragment davon verwendet.

**7.** Verfahren nach Anspruch 5 oder 6,
**dadurch gekennzeichnet,**
daß man chimäre Antikörper, die die variablen Regionen eines monoklonalen Maus-Antikörpers der gewünschten Spezifität, sowie die konstanten Regionen des Fab-Teils und den Fc-Teil eines monoklonalen humanen Antikörpers enthalten, oder Fragmente davon verwendet.

**8.** Verfahren nach einem der Ansprüche 5 bis 7,
**dadurch gekennzeichnet,**
daß man als Markierung eine Enzymmarkierung verwendet.

**9.** Verwendung von chimären monoklonalen Antikörpern, welche aus humanen Antikörpern bestehen, deren variable Regionen ganz oder teilweise durch die entsprechenden Teile von nicht-humanen monoklonalen Antikörpern der gewünschten Spezifität ersetzt sind, oder von Fragmenten davon zur Vermeidung von falsch-positiven Signalen durch unspezifische Bindung zwischen Testantikörpern und heterophilen Antikörpern bei "Sandwich"-Immunoassays zur quantitativen Bestimmung von Substanzen in Blut oder Serum von Patienten.

**10.** Verwendung nach Anspruch 9,
**dadurch gekennzeichnet,**
daß man einen chimären Antikörper, der die variablen Regionen eines monoklonalen Maus-Antikörpers der gewünschten Spezifität, sowie die konstanten Regionen des Fab-Teils und den Fc-Teil eines monoklonalen humanen Antikörpers enthält, oder ein Fragment davon verwendet.

11. Verwendung nach Anspruch 9,
**dadurch gekennzeichnet**,
daß man einen chimären monoklonalen Antikörper, der die hypervariablen Regionen eines monoklonalen Maus-Antikörpers der gewünschten Spezifität und den Fc-Teil, die konstanten Regionen des Fab-Teils sowie die framework-Bereiche aus den variablen Regionen eines humanen monoklonalen Antikörpers enthält, oder ein Fragment davon verwendet.

## Claims

1. Use of at least one chimeric monoclonal antibody which is composed of a human antibody, the variable region of which is completely or partially replaced by the corresponding parts of a non-human monoclonal antibody of the desired specificity or of a fragment thereof in a sandwich immunoassay for the quantitative immunological determination of substances in the blood or serum of patients, with the exception of immunoassays for the determination of CEA.

2. Use as claimed in claim 1,
**wherein**
the diagnostic test is carried out as an immunoradiometric (IRMA) or immuno-enzymatic (IEMA) assay.

3. Use as claimed in one of the claims 1 or 2,
**wherein**
a chimeric antibody, which contains the variable regions of a monoclonal mouse antibody of the desired specificity as well as the constant regions of the Fab part and the Fc part of a monoclonal human antibody, or a fragment thereof is used.

4. Use as claimed in one of the claims 1 or 2,
**wherein**
a chimeric monoclonal antibody, which contains the hypervariable regions of a monoclonal mouse antibody of the desired specificity and the Fc part, the constant regions of the Fab part as well as the framework regions from the variable regions of a human monoclonal antibody, or a fragment thereof is used.

5. Method for the diagnostic detection of substances, with the exception of CEA, in the serum or blood of patients by binding of two monoclonal antibodies $R_1$ and $R_2$ directed against the substance, of which the first antibody $R_1$ is capable of binding to a solid phase or is already bound and the second antibody $R_2$ carries a label, binding of the antibody $R_1$ to the solid phase if desired, separation of the complex formed of $R_1$, substance and $R_2$ which is bound to the solid phase and detection via the labelling of the antibody $R_2$,
**wherein**
a chimeric monoclonal antibody which is composed of a human antibody, the variable region of which is completely or partially replaced by the corresponding parts of a non-human monoclonal antibody of the desired specificity or a fragment thereof is used as at least one of the antibodies $R_1$ or $R_2$.

6. Method as claimed in claim 5,
**wherein**
a chimeric monoclonal antibody or a fragment thereof is used for both antibodies $R_1$ and $R_2$.

7. Method as claimed in claim 5 or 6,
**wherein**
chimeric antibodies, which contain the variable regions of a monoclonal mouse antibody of the desired specificity as well as the constant regions of the Fab part and the Fc part of a monoclonal human antibody, or fragments thereof are used.

8. Method as claimed in one of the claims 5 to 7,
**wherein**
an enzyme label is used as the label.

9. Use of chimeric monoclonal antibodies which are composed of human antibodies, the variable regions of which are replaced completely or partially by the corresponding parts of non-human monoclonal antibodies of the desired specificity, or by fragments thereof in order to avoid false positive signals by unspecific binding between test antibodies and heterophilic antibodies in "sandwich" immunoassays for the quantitative determination of substances in the blood or serum of patients.

10. Use as claimed in claim 9,
**wherein**
a chimeric monoclonal antibody which contains the variable regions of a monoclonal mouse antibody of the desired specificity, as well as the constant regions of the Fab part and the Fc part of a monoclonal human antibody, or a fragment thereof is used.

11. Use as claimed in claim 9,
**wherein**
a chimeric monoclonal antibody which contains the hypervariable regions of a monoclonal mouse antibody of the desired specificity and the Fc part, the constant regions of the Fab part, as well as the framework regions from the variable regions of a human monoclonal antibody, or a fragment thereof is used.

## Revendications

1. Utilisation d'au moins un anticorps monoclonal chimérique qui consiste en un anticorps humain dont la région variable est remplacée totalement ou en partie par les parties correspondantes d'un anticorps monoclonal non humain de spécificité souhaitée, ou d'un fragment de celui-ci dans un immunodosage "sandwich" pour la détermination immunologique quantitative de substances dans le sang ou le sérum de patients, à l'exception des immunodosages pour la détermination de CEA.

2. Utilisation selon la revendication 1, caractérisée en ce que le test diagnostique est réalisé sous forme de dosage immunoradiométrique (IRMA) ou immunoenzymatique (IEMA).

3. Utilisation selon l'une des revendications 1 ou 2, caractérisée en ce que l'on utilise un anticorps chimérique qui contient les régions variables d'un anticorps monoclonal de souris de spécificité souhaitée, ainsi que les régions constantes de la partie Fab et la partie Fc d'un anticorps monoclonal humain, ou un fragment de celui-ci.

4. Utilisation selon l'une des revendications 1 ou 2, caractérisée en ce que l'on utilise un anticorps monoclonal chimérique qui contient les régions hypervariables d'un anticorps monoclonal de souris de spécificité souhaitée et la partie Fc, les régions constantes de la partie Fab ainsi que les domaines de framework provenant des régions variables d'un anticorps monoclonal humain, ou un fragment de celui-ci.

5. Procédé pour la mise en évidence diagnostique de substances, à l'exception de CEA, dans le sérum ou le sang de patients par liaison de deux anticorps monoclonaux $R_1$ et $R_2$ dirigés contre la substance, parmi lesquels l'anticorps $R_1$ est capable de se lier ou est déjà lié à une phase solide et le second anticorps $R_2$ porte un marqueur, éventuellement liaison de l'anticorps $R_1$ à la phase solide, séparation du complexe lié à la phase solide formé constitué par $R_1$, la substance et $R_2$ et mise en évidence par le biais du marqueur de l'anticorps $R_2$, caractérisé en ce que l'on utilise pour au moins l'un des anticorps $R_1$ ou $R_2$ un anticorps monoclonal chimérique qui consiste en un anticorps humain dont la région variable est remplacée totalement ou en partie par les parties correspondantes d'un anticorps monoclonal non humain de spécificité souhaitée, ou un fragment de celui-ci.

6. Procédé selon la revendication 5, caractérisé en ce que l'on utilise pour les deux anticorps $R_1$ et $R_2$ des anticorps monoclonaux chimériques on un fragment de ceux-ci.

7. Procédé selon la revendication 5 ou 6, caractérisé en ce que l'on utilise des anticorps chimériques qui contiennent les régions variables d'un anticorps monoclonal de souris de spécificité souhaitée, ainsi que les régions constantes de la partie Fab et la partie Fc d'un anticorps monoclonal humain, ou des fragments de ceux-ci.

8. Procédé selon l'une des revendications 5 à 7, caractérisé en ce que l'on utilise comme marqueur un marqueur enzymatique.

**9.** Utilisation d'anticorps monoclonaux chimériques qui consistent en anticorps humains dont les régions variables sont remplacées totalement ou en partie par les parties correspondantes d'anticorps monoclonaux non humains de spécificité souhaitée, ou de fragments de ceux-ci pour éviter des signaux faussement positifs par liaison non spécifique entre des anticorps de test et des anticorps hétérophiles lors d'immunodosages "sandwich" pour la détermination quantitative de substances dans le sang ou le sérum de patients.

**10.** Utilisation selon la revendication 9, caractérisée en ce que l'on utilise un anticorps chimérique qui contient les régions variables d'un anticorps monoclonal de souris de spécificité souhaitée ainsi que les régions constantes de la partie Fab et la partie Fc d'un anticorps monoclonal humain, ou un fragment de celuici.

**11.** Utilisation selon la revendication 9, caractérisée en ce que l'on utilise un anticorps monoclonal chimérique qui contient les régions hypervariables d'un anticorps monoclonal de souris de spécificité souhaitée et la partie Fc, les régions constantes de la partie Fab ainsi que les domaines de framework provenant des régions variables d'un anticorps monoclonal humain, ou un fragment de celui-ci.

FIG.1

```
  1  GATGTTGTGATGACCCAGACTCCACTCACTTTGTCGGTTACCATTGGACAACCAGCCTCC
     AspValValMetThrGlnThrProLeuThrLeuSerValThrIleGlyGlnProAlaSer

 61  ATCTCTTGCAAGTCAAGTCAGAGCCTCTTAGATAGTGATGGAAGGACATATTTGAATTGG
     IleSerCysLysSerSerGlnSerLeuLeuAspSerAspGlyArgThrTyrLeuAsnTrp

121  TTGTTACAGAGGCCAGGCCAGTCTCCAAAGCGCCTAATCTATCTGGTGTCTAAACTGGAC
     LeuLeuGlnArgProGlyGlnSerProLysArgLeuIleTyrLeuValSerLysLeuAsp

181  TCTGGAGTCCCTGACAGGTTCACTGGCAGTGGATCAGGGACAGATTTCATACTGAAAATT
     SerGlyValProAspArgPheThrGlySerGlySerGlyThrAspPheIleLeuLysIle

241  AGCAGAGTGGAGGCTGAGGATTTGGGAGCTTATTATTGCTGGCAAGGTACACATTTTCCT
     SerArgValGluAlaGluAspLeuGlyAlaTyrTyrCysTrpGlnGlyThrHisPhePro

301  CAGACNTTCGGTGGAGGCACCAAGCTGGAAATCAAACGGGCTGATGCTGCACCAACTGTA
     GlnThrPheGlyGlyGlyThrLysLeuGluIleLysArgAlaAspAlaAlaProThrVal

361  TCCATCTTCCCACCATCCAGTGAGCAGTTAACATCTGGAGGTGCCTCAGTCGTGTGCTTC
     SerIlePheProProSerSerGluGlnLeuThrSerGlyGlyAlaSerValValCysPhe

421  TTGAACAACTTCTACCCCAAAGACATCAATGTCAAGTGGAAGATTGATGGCAGTGAACGA
     LeuAsnAsnPheTyrProLysAspIleAsnValLysTrpLysIleAspGlySerGluArg

481  CAAAATGGCGTCCTGAACAGTTGGACTGATCAGGACAGCAAAGACAGCACCTACAGCATG
     GlnAsnGlyValLeuAsnSerTrpThrAspGlnAspSerLysAspSerThrTyrSerMet

541  AGCAGCACCCTCACGTTGACCAAGGACGAGTATGAACGACATAACAGCTATACCTGTGAG
     SerSerThrLeuThrLeuThrLysAspGluTyrGluArgHisAsnSerTyrThrCysGlu

601  GCCACTCACAAGACATCAACTTCACCCATTGTCAAGAGCTTCAACAGGAATGAGTGTTAG
     AlaThrHisLysThrSerThrSerProIleValLysSerPheAsnArgAsnGluCysEnd
```

# FIG.2

```
   1  GATGTGCAGCTTCAGGAGTCAGGACCTGACCTGGTGAAACCTTCTCAGTCACTTTCACTC
      AspValGlnLeuGlnGluSerGlyProAspLeuValLysProSerGlnSerLeuSerLeu

  61  ACTTGCACTGTCACTGGCTACCCCATCACCAGTGGTTATACCTGGCACTGGATCCGGCAG
      ThrCysThrValThrGlyTyrProIleThrSerGlyTyrThrTrpHisTrpIleArgGln

 121  TTTCCAGGAAACTGTCTGGAATGGATGGGCTACATGCACTACAATGGTAGCACTAACTAC
      PheProGlyAsnCysLeuGluTrpMetGlyTyrMetHisTyrAsnGlySerThrAsnTyr

 181  AACCCATCTCTCAAAAGTCGAATCTCTATCACTCGAGACACATCCAAGAACCAGTTCTTC
      AsnProSerLeuLysSerArgIleSerIleThrArgAspThrSerLysAsnGlnPhePhe

 241  CTGCAGTTGAATTCTGTGACTACTGAGGACACAGCCACATATTACTGTGAATTCTCCTCT
      LeuGlnLeuAsnSerValThrThrGluAspThrAlaThrTyrTyrCysGluPheSerSer

 301  ATTATATGGGACTACTGGGGTCAAGGAACCTCAGTCACCGTCTCCTCAGCCAAAACGACA
      IleIleTrpAspTyrTrpGlyGlnGlyThrSerValThrValSerSerAlaLysThrThr

 361  CCCCCATCTGTCTATCCACTGGCCCCTGGATCTGCTGCCCAAACTAACTCCATGGTGACC
      ProProSerValTyrProLeuAlaProGlySerAlaAlaGlnThrAsnSerMetValThr

 421  CTGGGATGCCTGGTCAAGGGCTATTTCCCTGAGCCAGTGACAGTGACCTGGAACTCTGGA
      LeuGlyCysLeuValLysGlyTyrPheProGluProValThrValThrTrpAsnSerGly

 481  TCCCTGTCCAGCGGTGTGCACACCTTCCCAGCTGTCCTGCAGTCTGACCTCTACACTCTG
      SerLeuSerSerGlyValHisThrPheProAlaValLeuGlnSerAspLeuTyrThrLeu

 541  AGCAGCTCAGTGACTGTCCCCTCCAGCACCTGGCCCAGCGAGACCGTCACCTGCAACGTT
      SerSerSerValThrValProSerSerThrTrpProSerGluThrValThrCysAsnVal

 601  GCCCACCCGGCCAGCAGCACCAAGGTGGACAAGAAAATTGTGCCCAGGGATTGTGGTTGT
      AlaHisProAlaSerSerThrLysValAspLysLysIleValProArgAspCysGlyCys

 661  AAGCCTTGCATATGTACAGTCCCAGAAGTATCATCTGTCTTCATCTTCCCCCCAAAGCCC
      LysProCysIleCysThrValProGluValSerSerValPheIlePheProProLysPro

 721  AAGGATGTGCTCACCATTACTCTGACTCCTAAGGTCACGTGTGTTGTGGTAGACATCAGC
      LysAspValLeuThrIleThrLeuThrProLysValThrCysValValValAspIleSer

 781  AAGGATGATCCCGAGGTCCAGTTCAGCTGGTTTGTAGATGATGTGGAGGTGCACACAGCT
      LysAspAspProGluValGlnPheSerTrpPheValAspAspValGluValHisThrAla

 841  CAGACGCAACCCCGGGAGGAGCAGTTCAACAGCACTTTCCGCTCAGTCAGTGAACTTCCC
      GlnThrGlnProArgGluGluGlnPheAsnSerThrPheArgSerValSerGluLeuPro

 901  ATCATGCACCAGGACTGGCTCAATGGCAAGGAGTTCAAATGCAGGGTCAACAGTGCAGCT
      IleMetHisGlnAspTrpLeuAsnGlyLysGluPheLysCysArgValAsnSerAlaAla

 961  TTCCCTGCCCCCATCGAGAAAACCATCTCCAAAACCAAAGGCAGACCGAAGGCTCCACAG
      PheProAlaProIleGluLysThrIleSerLysThrLysGlyArgProLysAlaProGln

1021  GTGTACACCATTCCACCTCCCAAGGAGCAGATGGCCAAGGATAAAGTCAGTCTGACCTGC
      ValTyrThrIleProProProLysGluGlnMetAlaLysAspLysValSerLeuThrCys
```

FIG.2

(Fortsetzung)

```
1081  ATGATAACAGACTTCTTCCCTGAAGACATTACTGTGGAGTGGCAGTGGAATGGGCAGCCA
      MetIleThrAspPhePheProGluAspIleThrValGluTrpGlnTrpAsnGlyGlnPro

1141  GCGGAGAACTACAAGAACACTCAGCCCATCATGAACACGAATGGCTCTTACTTCGTCTAC
      AlaGluAsnTyrLysAsnThrGlnProIleMetAsnThrAsnGlySerTyrPheValTyr

1201  AGCAAGCTCAATGTGCAGAAGAGCAACTGGGAGGCAGGAAATACTTTCACCTGCTCTGTG
      SerLysLeuAsnValGlnLysSerAsnTrpGluAlaGlyAsnThrPheThrCysSerVal

1261  TTACATGAGGGCCTGCACAACCACCATACTGAGAAGAGCCTCTCCCACTCTCCTGGTAAA
      LeuHisGluGlyLeuHisAsnHisHisThrGluLysSerLeuSerHisSerProGlyLys

1321  TGATCCCAGTGTCCTTGGAGCCCTCTGGTCCTACAGGACTCTGACACCTACCTCCACCCC
      EndSerGlnCysProTrpSerProLeuValLeuGlnAspSerAspThrTyrLeuHisPro

1381  TCCCTGTATAAATAA  1395
      SerLeuTyrLysEnd
```

# FIG.3

```
  1  GATATCGTGATGACCCAGACTCCACTCACTTTGTCGGTTACCATTGGACAACCAGCCTCC
     AspIleValMetThrGlnThrProLeuThrLeuSerValThrIleGlyGlnProAlaSer

 61  ATCTCTTGCAAGTCAAGTCAGAGCCTCTTAGATAGTGATGGAAGGACATATTTGAATTGG
     IleSerCysLysSerSerGlnSerLeuLeuAspSerAspGlyArgThrTyrLeuAsnTrp

121  TTGTTACAGAGGCCAGGCCAGTCTCCAAAGCGCCTAATCTATCTGGTGTCTAAACTGGAC
     LeuLeuGlnArgProGlyGlnSerProLysArgLeuIleTyrLeuValSerLysLeuAsp

181  TCTGGAGTCCCTGACAGGTTCACTGGCAGTGGATCAGGGACAGATTTCATACTGAAAATT
     SerGlyValProAspArgPheThrGlySerGlySerGlyThrAspPheIleLeuLysIle

241  AGCAGAGTGGAGGCTGAGGATTTGGGAGCTTATTATTGCTGGCAAGGTACACATTTTCCT
     SerArgValGluAlaGluAspLeuGlyAlaTyrTyrCysTrpGlnGlyThrHisPhePro

301  CAGACNTTCGGTGGAGGCACCAAGCTCGAGATCAAACGAACTGTGGCTGCACCATCTGTC
     GlnThrPheGlyGlyGlyThrLysLeuGluIleLysArgThrValAlaAlaProSerVal

361  TTCATCTTCCCGCCATCTGATGAGCAGTTGAAATCTGGAACTGCCTCTGTTGTGTGCCTG
     PheIlePheProProSerAspGluGlnLeuLysSerGlyThrAlaSerValValCysLeu

421  CTGAATAACTTCTATCCCAGAGAGGCCAAAGTACAGTGGAAGGTGGATAACGCCCTCCAA
     LeuAsnAsnPheTyrProArgGluAlaLysValGlnTrpLysValAspAsnAlaLeuGln

481  TCGGGTAACTCCCAGGAGAGTGTCACAGAGCAGGAGAGCAAGGACAGCACCTACAGCCTC
     SerGlyAsnSerGlnGluSerValThrGluGlnGluSerLysAspSerThrTyrSerLeu

541  AGCAGCACCCTGACGCTGAGCAAAGCAGACTACGAGAAACACAAAGTCTACGCCTGCGAA
     SerSerThrLeuThrLeuSerLysAlaAspTyrGluLysHisLysValTyrAlaCysGlu

601  GTCACCCATCAGGGCCTGAGCTCGCCCGTCACAAAGAGCTTCAACAGGGGAGAGTGTTAG
     ValThrHisGlnGlyLeuSerSerProValThrLysSerPheAsnArgGlyGluCysEnd
```

FIG. 4

```
  1  GAGGTCCAGCTGCAAGAGTCAGGACCTGACCTGGTGAAACCTTCTCAGTCACTTTCACTC 6
     GluValGlnLeuGlnGluSerGlyProAspLeuValLysProSerGlnSerLeuSerLeu

 61  ACTTGCACTGTCACTGGCTACCCCATCACCAGTGGTTATACCTGGCACTGGATCCGGCAG 1
     ThrCysThrValThrGlyTyrProIleThrSerGlyTyrThrTrpHisTrpIleArgGln

121  TTTCCAGGAAACTGTCTGGAATGGATGGGCTACATGCACTACAATGGTAGCACTAACTAC 1
     PheProGlyAsnCysLeuGluTrpMetGlyTyrMetHisTyrAsnGlySerThrAsnTyr

181  AACCCATCTCTCAAAAGTCGAATCTCTATCACTCGAGACACATCCAAGAACCAGTTCTTC 2
     AsnProSerLeuLysSerArgIleSerIleThrArgAspThrSerLysAsnGlnPhePhe

241  CTGCAGTTGAATTCTGTGACTACTGAGGACACAGCCACATATTACTGTGAATTCTCCTCT 3
     LeuGlnLeuAsnSerValThrThrGluAspThrAlaThrTyrTyrCysGluPheSerSer

301  ATTATATGGGACTACTGGGGTCAAGGAACCTCAGTCACCGTCTCTGCAGCCTCCACCAAG 3
     IleIleTrpAspTyrTrpGlyGlnGlyThrSerValThrValSerAlaAlaSerThrLys

361  GGCCCATCGGTCTTCCCCCTGGCACCCTCCTCCAAGAGCACCTCTGGGGGCACAGCGGCC 4
     GlyProSerValPheProLeuAlaProSerSerLysSerThrSerGlyGlyThrAlaAla

421  CTGGGCTGCCTGGTCAAGGACTACTTCCCCGAACCGGTGACGGTGTCGTGGAACTCAGGC 4
     LeuGlyCysLeuValLysAspTyrPheProGluProValThrValSerTrpAsnSerGly

481  GCCCTGACCAGCGGCGTGCACACCTTCCCGGCTGTCCTACAGTCCTCAGGACTCTACTCC 5
     AlaLeuThrSerGlyValHisThrPheProAlaValLeuGlnSerSerGlyLeuTyrSer

541  CTCAGCAGCGTGGTGACCGTGCCCTCCAGCAGCTTGGGCACCCAGACCTACATCTGCAAC 6
     LeuSerSerValValThrValProSerSerSerLeuGlyThrGlnThrTyrIleCysAsn

601  GTGAATCACAAGCCCAGCAACACCAAGGTGGACAAGAAAGTTGAGCCCAAATCTTGTGAC 6
     ValAsnHisLysProSerAsnThrLysValAspLysLysValGluProLysSerCysAsp

661  AAAACTCACACATGCCCACCGTGCCCAGCACCTGAACTCCTGGGGGGACCGTCAGTCTTC 7
     LysThrHisThrCysProProCysProAlaProGluLeuLeuGlyGlyProSerValPhe

721  CTCTTCCCCCCAAAACCCAAGGACACCCTCATGATCTCCCGGACCCCTGAGGTCACATGC 7
     LeuPheProProLysProLysAspThrLeuMetIleSerArgThrProGluValThrCys

781  GTGGTGGTGGACGTGAGCCACGAAGACCCTGAGGTCAAGTTCAACTGGTACGTGGACGGC 8
     ValValValAspValSerHisGluAspProGluValLysPheAsnTrpTyrValAspGly

841  GTGGAGGTGCATAATGCCAAGACAAAGCCGCGGGAGGAGCAGTACAACAGCACGTACCGG 9
     ValGluValHisAsnAlaLysThrLysProArgGluGluGlnTyrAsnSerThrTyrArg

901  GTGGTCAGCGTCCTCACCGTCCTGCACCAGGACTGGCTGAATGGCAAGGAGTACAAGTGC 9
     ValValSerValLeuThrValLeuHisGlnAspTrpLeuAsnGlyLysGluTyrLysCys

961  AAGGTCTCCAACAAAGCCCTCCCAGCCCCCATCGAGAAAACCATCTCCAAAGCCAAAGGG 1
     LysValSerAsnLysAlaLeuProAlaProIleGluLysThrIleSerLysAlaLysGly
```

FIG.4

(Fortsetzung)

```
1021  CAGCCCCGAGAACCACAGGTGTACACCCTGCCCCCATCCCGGGATGAGCTGACCAAGAAC 1
      GlnProArgGluProGlnValTyrThrLeuProProSerArgAspGluLeuThrLysAsn

1081  CAGGTCAGCCTGACCTGCCTGGTCAAAGGCTTCTATCCCAGCGACATCGCCGTGGAGTGG 1
      GlnValSerLeuThrCysLeuValLysGlyPheTyrProSerAspIleAlaValGluTrp

1141  GAGAGCAATGGGCAGCCGGAGAACAACTACAAGACCACGCCTCCCGTGCTGGACTCCGAC 1
      GluSerAsnGlyGlnProGluAsnAsnTyrLysThrThrProProValLeuAspSerAsp

1201  GGCTCCTTCTTCCTCTACAGCAAGCTCACCGTGGACAAGAGCAGGTGGCAGCAGGGGAAC 1
      GlySerPhePheLeuTyrSerLysLeuThrValAspLysSerArgTrpGlnGlnGlyAsn

1261  GTCTTCTCATGCTCCGTGATGCATGAGGCTCTGCACAACCACTACACGCAGAAGAGCCTC 1
      ValPheSerCysSerValMetHisGluAlaLeuHisAsnHisTyrThrGlnLysSerLeu

1321  TCCCTGTCTCCGGGTAAATGA  1341
      SerLeuSerProGlyLysEnd
```

# FIG. 5

Vektor p11196

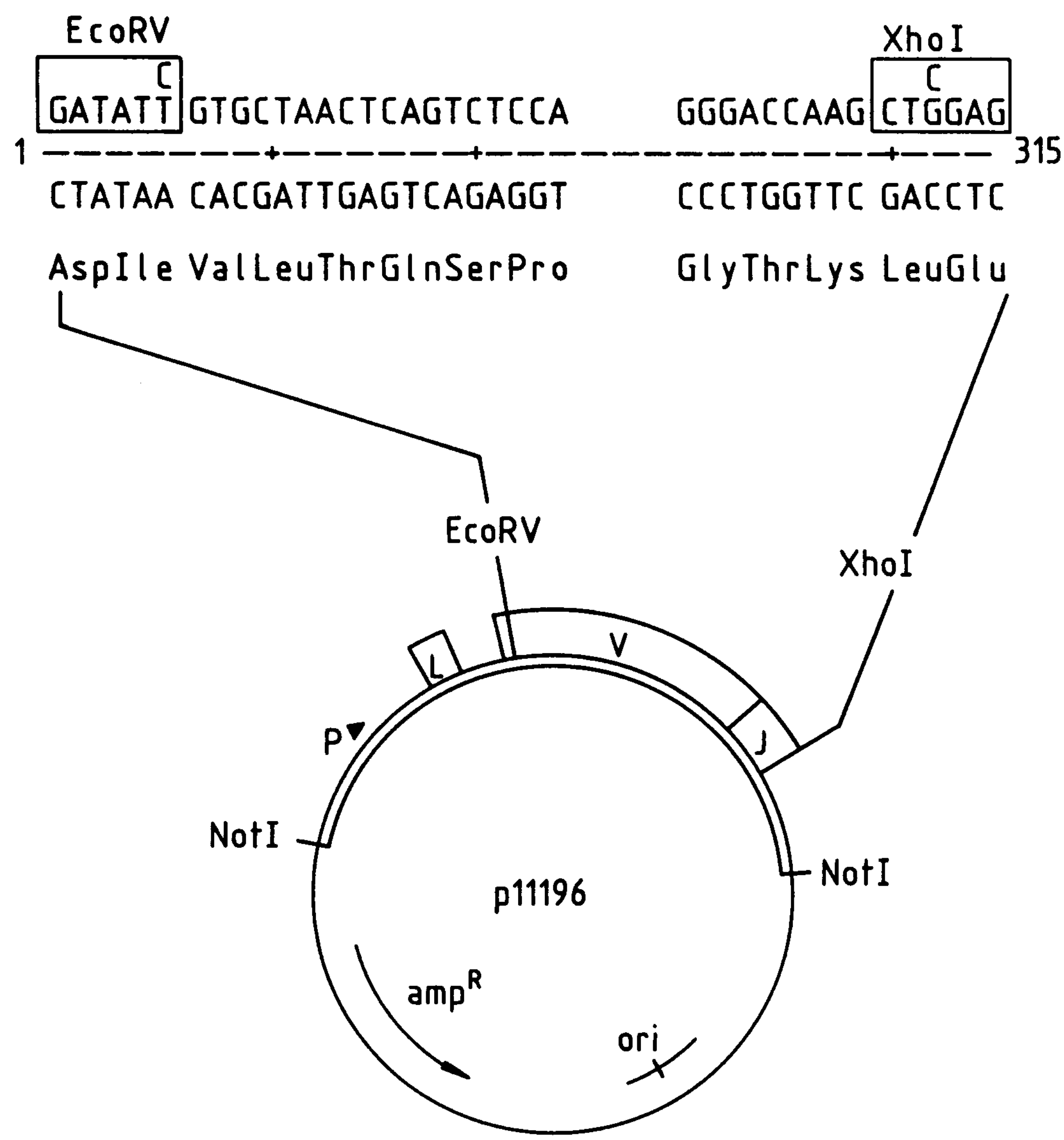

FIG.6

Vektor p11197

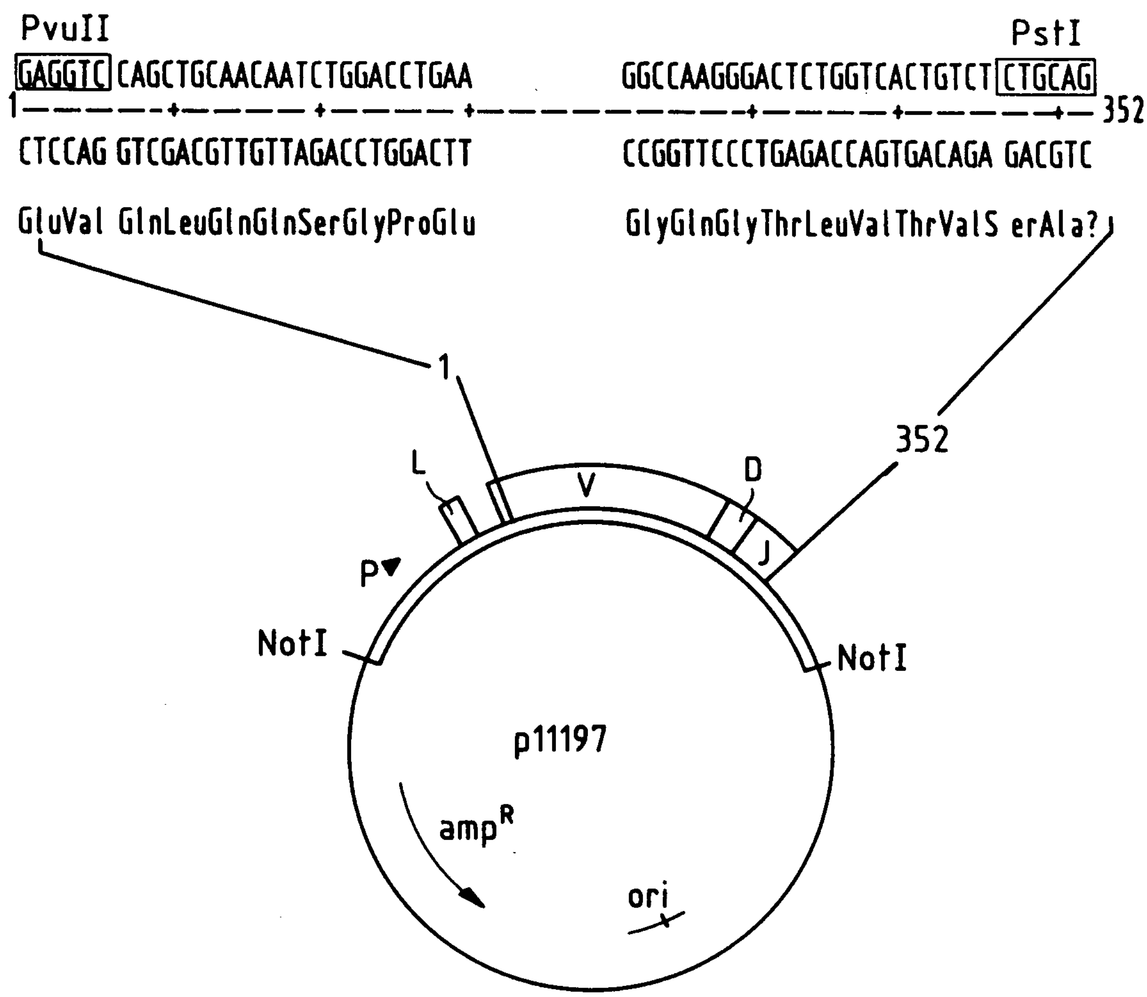

FIG.7

Vektor p10195

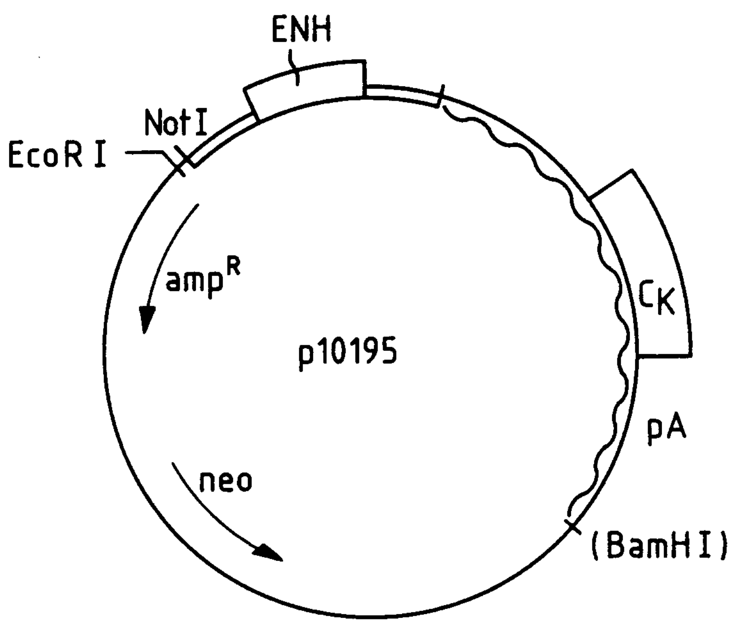

FIG.8

Vektor p11201

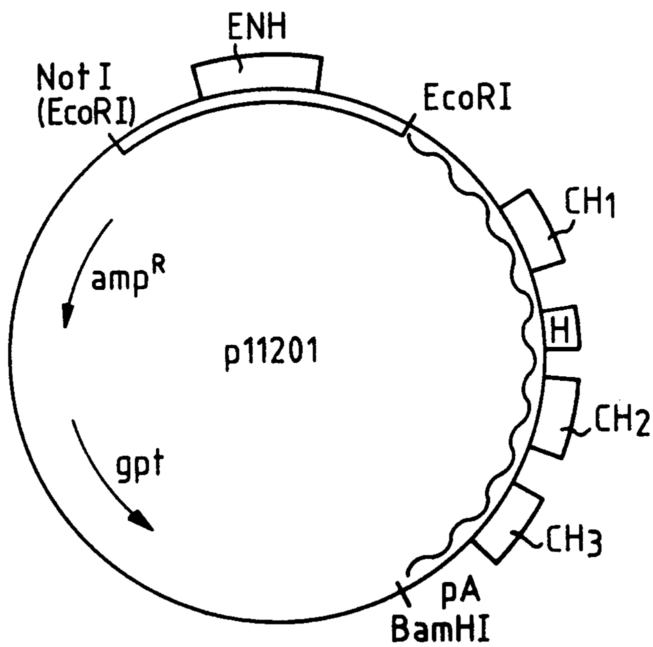

FIG. 9

Übersicht über die Klonierschritte der K-Konstruktion

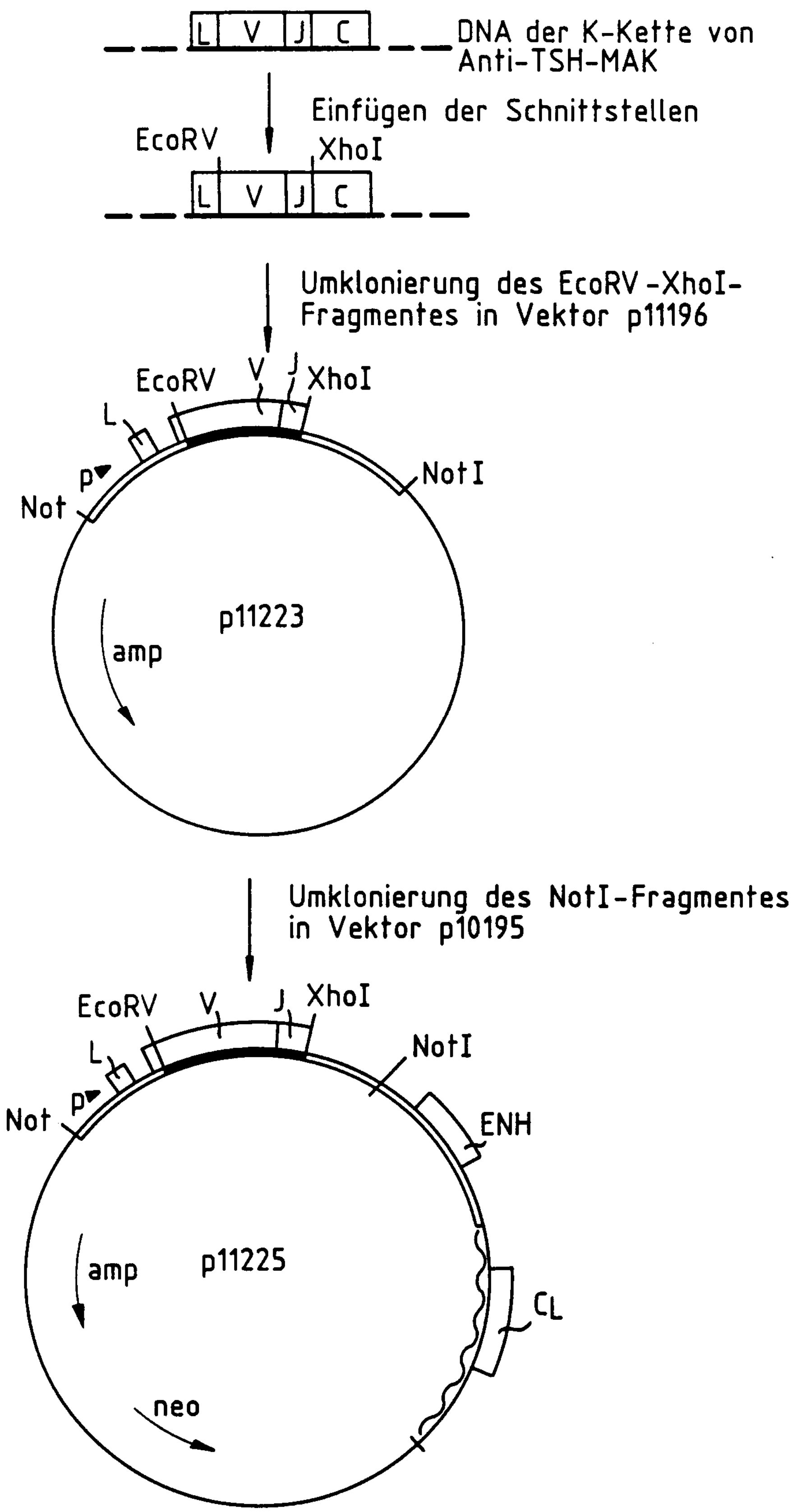

# FIG.10

Übersicht über die Klonierschritte der γ-Konstruktion

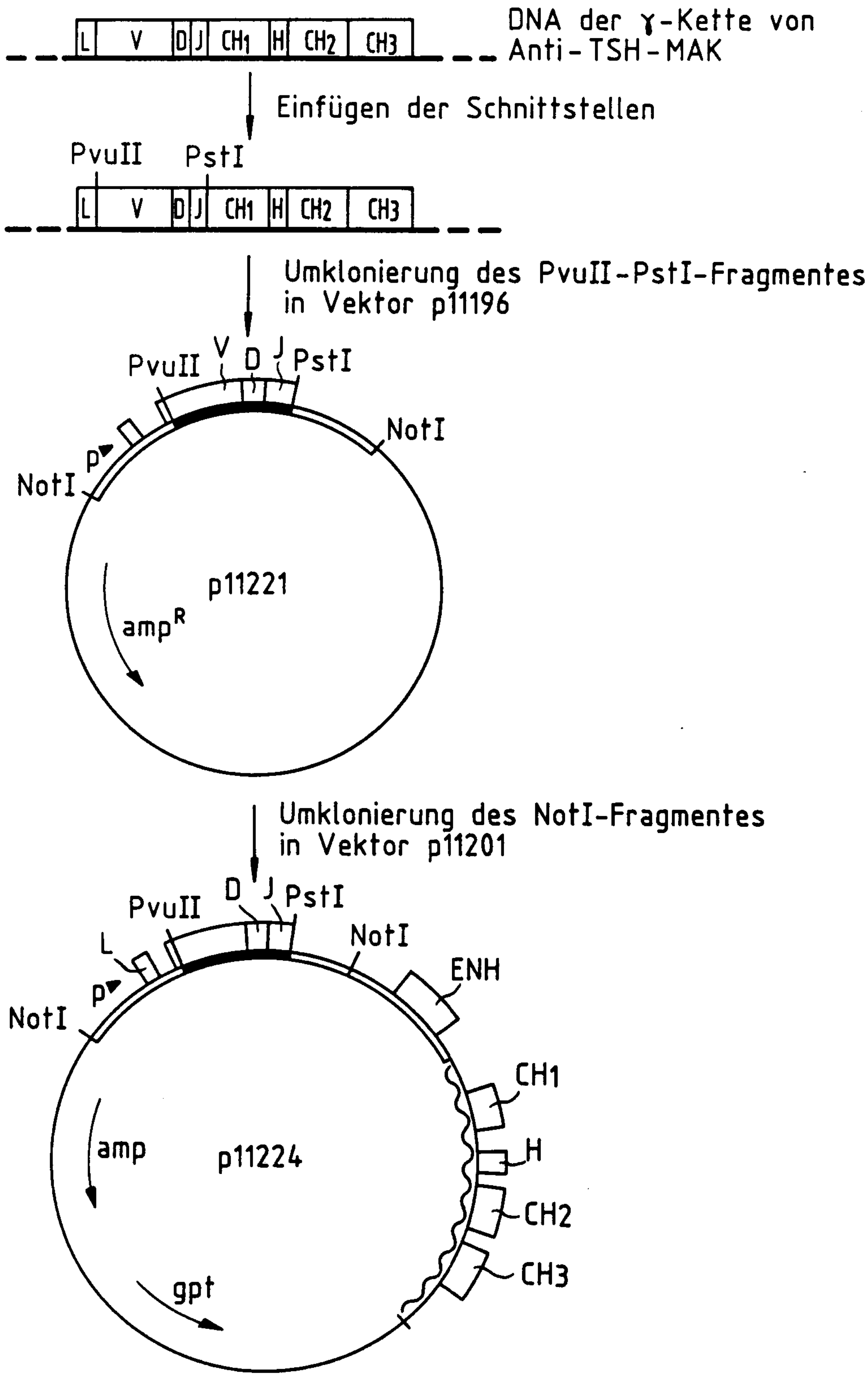

FIG. 11

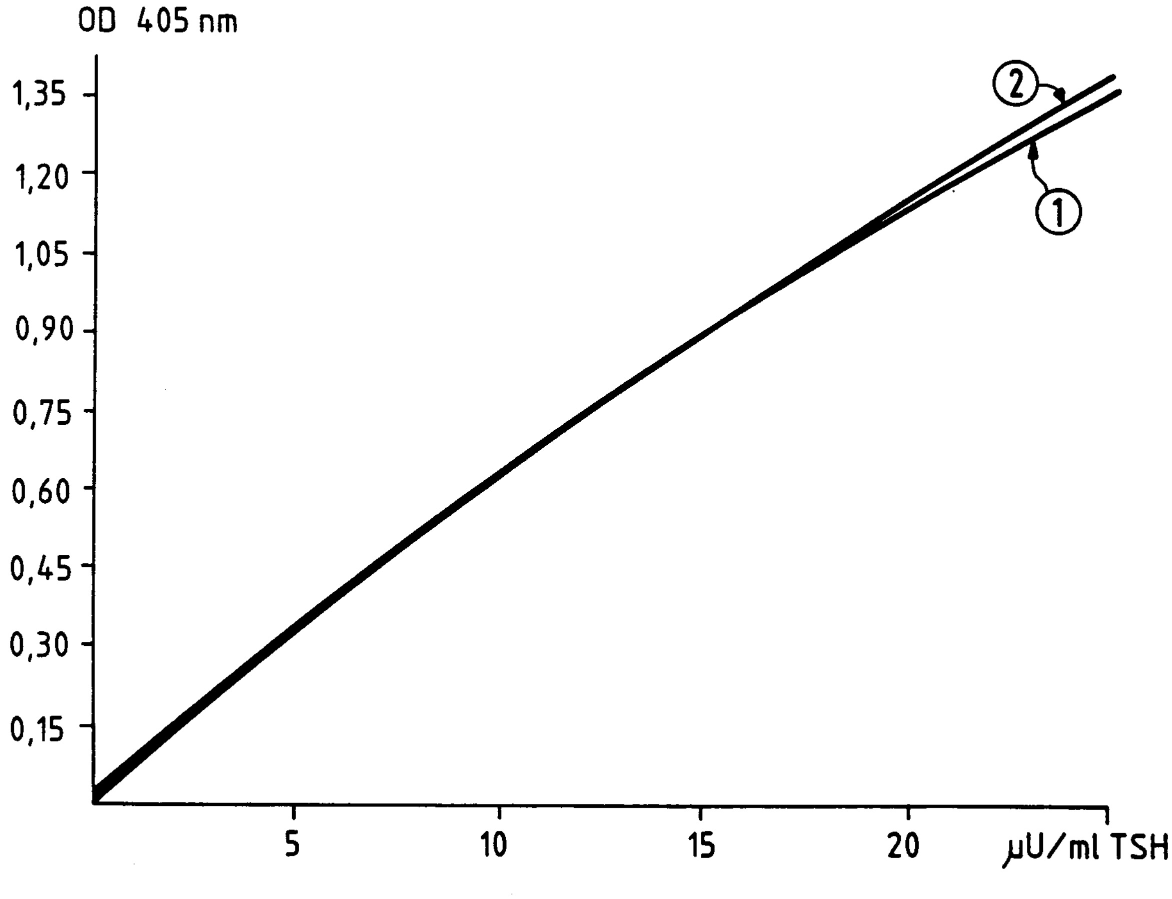
OD 405 nm
1,35
1,20
1,05
0,90
0,75
0,60
0,45
0,30
0,15
5
10
15
20
µU/ml TSH
2
1